# EUROPEAN PATENT APPLICATION

(11) **EP 4 374 796 A1**
(43) Date of publication of application: **29.05.2024**
(21) Application number: 22845438.5
(22) Date of filing: 22.07.2022
(51) Int. Cl.: A61B 17/12

(54) **MEDICAL DEVICE**

(30) Priority: 22.07.2021 CN 202110839950; 22.07.2021 CN 202110833154; 22.07.2021 CN 202110833159
(71) Applicant: Lifetech Scientific (Shenzhen) Co., Ltd., Shenzhen, Guangdong 518063 (CN)
(72) Inventor: LI, Anning, Shenzhen, Guangdong 518063 (CN); LIU, Jianyong, Shenzhen, Guangdong 518063 (CN); ZHU, Wancheng, Shenzhen, Guangdong 518063 (CN)
(74) Representative: Prinz & Partner mbB
(86) International application number: PCT/CN2022/107345
(87) International publication number: WO 2023/001275

(57) **Abstract**

The present invention relates to a medical device, including a fixing part, and having a compressed state and a deployed state after self-expansion, where the fixing part is provided with at least one anchoring member configured to be clamped in a gap on body tissue after the medical device is implanted into the body tissue, so as to anchor the medical device in the body. The present invention prevents damage to human tissue by providing anchoring members that are clamped in the gap in the human tissue rather than piercing through the human tissue after the medical device is implanted into the human body, thereby further preventing wound damage from enlarging due to movement of the human tissue. Normal operation of the medical device is achieved while ensuring good anchoring ability.

## Description

### Technical Field

The embodiments relate to the technical field of medical devices, in particular to a medical device.

### Background Art

Medical devices, particularly medical implant devices, are generally required to remain at the implantation site for a period of time after implantation into the human body.

In recent years, 90% of the stroke caused by atrial fibrillation in patients with non-valvular atrial fibrillation originated from the left atrial appendage (LAA). It has been shown by clinical data that resection of LAA by a cardiac surgery may reduce the incidence of stroke in patients with atrial fibrillation, suggesting the danger of LAA in thromboembolism. Since LAA is a "den" of thrombus, the basis for thrombus formation in LAA may be eliminated by occluding the LAA ostium. In general, the use of a LAA occluder as a medical implant device to occlude the LAA ostium is an effective way to prevent stroke caused by atrial fibrillation.

In order to effectively occlude LAA, long-term implantation of the LAA occluder in LAA is required to achieve the occlusion effect. Therefore, the LAA occluder needs an anchoring structure to realize a stable long-term occlusion at the LAA ostium and avoid problems such as device embolization caused by its detachment.

In order to achieve the long-term stability of occlusion of the LAA occluder at the LAA ostium, a plurality of sharp-tipped anchoring structures, such as anchoring barbs or anchoring hooks, are generally provided at the supporting part of the LAA occluder (the interface between the LAA occluder and the atrial appendage wall) to pierce into the atrial appendage wall to realize the long-term stability of implantation. However, such sharp-tipped anchoring structures in the shape of anchoring barbs or anchoring hooks are likely to puncture the atrial appendage wall, causing complications such as pericardial effusion and endangering the lives of patients. Meanwhile, as the atrial appendage undergoes systolic and diastolic motion with the heart, a greater damage may be caused at the anchoring site due to the motion of the atrial appendage if the anchoring is performed by piercing. Such problems occur in other human implants as well, and therefore, a medical device is desired that has anchoring stability while avoiding the introduction of sharp-tipped anchoring structures.

### Summary of the Invention

In view of the above, it is necessary to provide a new medical device for the problem that the anchoring barb structures of medical devices in the prior art pierce through the human tissue.

A medical device includes a fixing part configured to fix the medical device at a predetermined position, where the fixing part is provided with at least one anchoring member configured to abut against a target cavity after the medical device is implanted at the predetermined position, so as to anchor the medical device at the predetermined position.

In one embodiment, the anchoring member includes at least one anchoring unit.

In one embodiment, the anchoring member further includes a connecting unit.

In one embodiment, the fixing part includes a cover membrane, and the connecting unit connects the anchoring unit to the cover membrane.

In one embodiment, the fixing part includes a mesh support structure, and the connecting unit connects the anchoring unit to the mesh support structure.

In one embodiment, the fixing part includes a supporting rod, and the connecting unit connects the anchoring unit to the supporting rod.

In one embodiment, the anchoring unit is positioned between the fixing part and the cover membrane, and the cover membrane at a position of the anchoring unit protrudes in a direction facing away from an axis of the medical device.

In one embodiment, the mesh support structure includes a supporting wire, the connecting unit is connected to the supporting wire by entanglement, and the connecting unit is fixedly connected to the supporting wire, or the connecting unit is slidable with respect to the supporting wire along a length direction of the supporting wire.

In one embodiment, a limit unit is further provided on the supporting wire to which the connecting unit is connected so as to limit a sliding range of the connecting unit on the supporting wire when the connecting unit is slidable with respect to the supporting wire along the length direction of the supporting wire.

In one embodiment, the mesh support structure includes a plurality of supporting wires, the anchoring unit is connected to the plurality of supporting wires by the connecting unit, and when the fixing part is in a radially compressed state, the anchoring unit is positioned at a medial side of the mesh support structure, or the anchoring unit is positioned at least partially in a gap between adjacent supporting wires.

In one embodiment, a plurality of anchoring units are provided on the fixing part, and the plurality of anchoring units are positioned on a plurality of cross-sections of the fixing part when the fixing part is in a deployed state; and/or the plurality of anchoring units are positioned on a plurality of cross-sections of the fixing part when the fixing part is in a radially compressed state.

In one embodiment, the anchoring member includes a connecting rod and a connecting block, a first channel is arranged on the fixing part which runs through medial and lateral sides of the fixing part, one end of the connecting rod is connected to the anchoring unit, and the other end is connected to the connecting block after passing through the first channel.

In one embodiment, the anchoring unit is rotatably connected to the connecting unit.

In one embodiment, the connecting unit includes an axially retractable rod having one end connected to the anchoring unit and the other end fixedly connected to the fixing part.

In one embodiment, the fixing part is provided with a receiving cavity that receives or partially receives the anchoring unit when the medical device is in a compressed state.

In one embodiment, the receiving cavity includes a first cavity that at least partially receives the anchoring unit when the medical device is in the compressed state and a second cavity that at least partially receives the connecting unit when the medical device is in the compressed state.

In one embodiment, the anchoring unit extends obliquely outward in a natural state, and an opening angle of the anchoring unit with respect to the fixing part in the natural state is 20°-60°.

In one embodiment, the anchoring member further includes a flexible connecting unit, at least one hole is arranged on the fixing part which runs through medial and lateral sides of the fixing part, one end of the flexible connecting unit is movably fixed to the fixing part after passing through the hole, and the anchoring unit is fixed to the flexible connecting unit.

In one embodiment, the fixing part includes a plurality of anchoring members, at least one of the anchoring members having a different opening angle and/or a different horizontal position from anchoring members adjacent thereto.

In one embodiment, the anchoring unit is provided with micro-barbs.

The medical device provided by the present invention prevents damage to human tissue by providing anchoring members that are clamped in the gap in the human tissue or abutting against the inner wall of the human tissue rather than piercing through the human tissue to realize anchoring after the medical device is implanted into the human body, thereby further preventing wound damage from enlarging due to movement of the human tissue. Normal operation of the medical device is achieved while ensuring good anchoring ability.

### Brief Description of the Drawings

FIG 1 is a structural diagram of a medical device in Embodiment 1 of the present invention;
FIG 2 is a structural diagram of an anchoring member of the medical device in Embodiment 1 of the present invention;
FIG 3 is a structural diagram of an anchoring member of a medical device in another embodiment of the present invention;
FIG 4 is a structural diagram of an anchoring member of a medical device in Embodiment 2 of the present invention;
FIG 5 is a structural diagram of an anchoring member of a medical device in Embodiment 3 of the present invention;
FIG 6 is a structural diagram of an anchoring member of a medical device in another embodiment of the present invention;
FIG 7 is a structural diagram of an anchoring member of a medical device in Embodiment 4 of the present invention;
FIG 8 is an exploded view of the anchoring member of the medical device in Embodiment 4 of the present invention;
FIG 9 is a structural diagram of an anchoring member of a medical device in Embodiment 5 of the present invention;
FIG 10 is a structural diagram of an anchoring member of a medical device in Embodiment 6 of the present invention;
FIG 11 is a structural diagram of the anchoring member of the medical device in Embodiment 6 of the present invention from another perspective;
FIG 12 is a structural diagram of an anchoring member of a medical device in Embodiment 7 of the present invention;
FIG 13 is a structural diagram of the anchoring member of the medical device in Embodiment 7 of the present invention from another perspective;
FIG 14 is a structural diagram showing application of an anchoring member of a medical device of the present invention to a first LAA occluder;
FIG 15 is a structural diagram showing application of an anchoring member of a medical device of the present invention to a second LAA occluder;
FIG 16 is a structural diagram showing application of an anchoring member of a medical device of the present invention to a third LAA occluder;
FIG 17 is a structural diagram showing application of an anchoring member of a medical device of the present invention to a fourth LAA occluder;
FIG 18 is a structural diagram of a medical device in Embodiment 8 of the present invention;
FIG 19 is a structural diagram of a main body of the medical device in Embodiment 8 of the present invention;
FIG 20 is a structural diagram of an anchoring member of the medical device in Embodiment 8 of the present invention;
FIG 21 is a structural diagram of an anchoring member of a medical device in another embodiment of the present invention;
FIG 22 is a working diagram of the anchoring member after implantation of the medical device in Embodiment 8 of the present invention;
FIG 23 is a structural diagram of an anchoring member of a medical device in Embodiment 9 of the present invention;
FIG 24 is a cross-sectional diagram of the anchoring member of the medical device in Embodiment 9 of the present invention;
FIG 25 is a structural diagram of an anchoring member of a medical device in another embodiment of the present invention;
FIG 26 is a structural diagram of an anchoring member of a medical device in Embodiment 10 of the present invention;
FIG 27 is a structural diagram of an anchoring member of a medical device in Embodiment 11 of the present invention;
FIG 28 is an exploded view of the anchoring member of the medical device in Embodiment 11 of the present invention;
FIG 29 is a structural diagram of an anchoring member in Embodiment 12 of the present invention from a first perspective;
FIG 30 is a structural diagram of the anchoring member in Embodiment 12 of the present invention from a second perspective;
FIG 31 is a structural diagram of the anchoring member in Embodiment 12 of the present invention from a third perspective;
FIG 32 is a schematic diagram showing an implantation state of a medical device in Embodiment 13 of the present invention;
FIG 33 is a structural diagram of the medical device in Embodiment 13 of the present invention;
FIG 34 is a structural diagram of an anchoring member of a medical device in Embodiment 14 of the present invention;
FIG 35 is a structural diagram of an anchoring member of a medical device in another embodiment of the present invention;
FIG 36 is a structural diagram of an anchoring member of a medical device in Embodiment 15 of the present invention;
FIG 37 is a structural diagram of an anchoring member of a medical device in another embodiment of the present invention; and
FIG 38 is a structural diagram of a medical device in Embodiment 16 of the present invention.

### Detailed Description of the Invention

In order that the objects, technical schemes, and advantages of the present invention become clearer, the present invention will be further illustrated in detail below with reference to the drawings and embodiments. It is to be understood that the particular embodiments described herein are illustrative only and are not intended to be limiting.

It is to be noted that in the field of interventional medical devices, an end of a medical device implanted in a human body or an animal which is close to the operator is generally called a "proximal end", and an end which is far away from the operator is called a "distal end", and therefore a "proximal end" and a "distal end" of any part of a medical device may be defined in accordance with this principle. An "axial" direction generally refers to a length direction of a medical device as it is delivered, and a "radial" direction generally refers to a direction of the medical device perpendicular to the "axial" direction thereof, and therefore an "axial" direction and a "radial" direction of any part of a medical device may be defined in accordance with this principle.

Hereinafter, the technical schemes of the present invention will be described in further detail with reference to specific embodiments.

### Embodiment 1

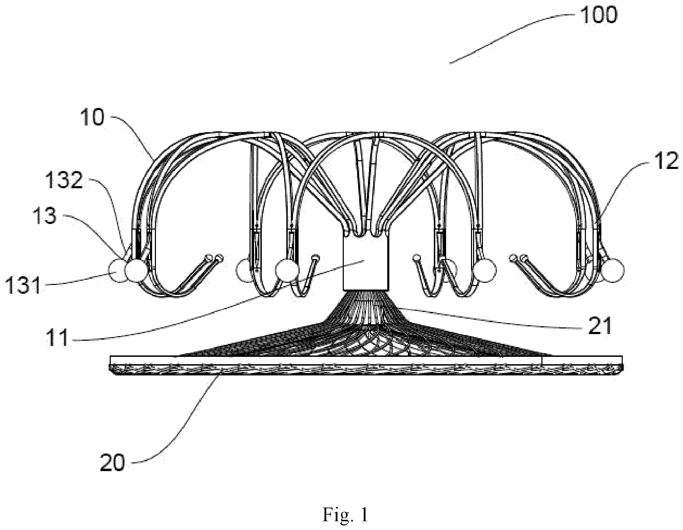

As shown in FIG 1, which is a structural diagram of a medical device 100 of Embodiment 1 of the present invention, the medical device 100 of this implementation includes a fixing part 10 and a sealing part 20 connected to the fixing part 10. The sealing part 20 and the fixing part 10 are spaced apart along an axial direction of the medical device 100. The sealing part 20 is positioned at a proximal end of the medical device 100, and the fixing part 10 is positioned at a distal end of the medical device 100. The medical device 100 has a compressed state as housed within a sheath to facilitate delivery and a deployed state as shown in FIG 1 after extending from a distal end of the sheath and self-expanding for deployment. The shape of the medical device 100 after release into the LAA cavity is identical or substantially identical to that of FIG 1.

The sealing part 20 may be formed by weaving a plurality of woven wires into a mesh tube, and then closing and fixing ends of the woven wires by a plug 21 at a distal end of the sealing part separately at two ends of the mesh tube. Then, the mesh tube is heat-set into a shape such as a disc shape, a cylindrical shape, or a plug shape, thereby obtaining a sealing part 20 for occluding the LAA ostium. At least one layer of film may be provided inside the sealing part 20 (not shown), the edge of which is fixed to the woven wires at the edge of the sealing part 20. The film serves to prevent blood flow from one side to the other side of the sealing part 20 so as to prevent blood flow between LAA and the left atrium.

The fixing part 10 includes a plug 11 of the fixing part and a plurality of supporting rods 12, and the plug 21 at the distal end of the sealing part is connected to the plug 11 of the fixing part via a connecting member 30. The supporting rods 12 of the fixing part 10 may be rods obtained by cutting a metal alloy tube or a polymer tube, or may be rods formed by weaving or winding woven wires.

As shown in FIG 1, proximal ends of the plurality of supporting rods 12 are connected to the plug 11 of the fixing part, and distal ends extend radially outward from a central end portion and turn toward the sealing part, thereby forming a support surface for contacting the LAA cavity wall and performing a support function. The supporting rod 12 may be provided with an anchoring member 13 which is generally arranged on the supporting rod 12 by welding, hot melting, entanglement, bonding, etc., without excluding that the anchoring member 13 is directly cut when the supporting rod 12 is formed by cutting.

In this embodiment, the anchoring member 13 includes an anchoring unit and a connecting unit, where the anchoring unit in this embodiment is preferably a protrusion 131, and the connecting unit is selected as a rod-shaped connecting member 132, where one end of the connecting member 132 is connected to the supporting rod 12, and the other end is connected to the protrusion 131. The protrusion 131 may have a spherical, hemispherical, drop-shaped, cylindrical, conical, polyhedral structure, etc. It is to be noted that the protrusion is not the only manifestation of the anchoring unit 121, and the anchoring unit 121 may be configured in a variety of structures, such as annular, umbrella-shaped, disc-shaped, and radial, as long as the protrusion 131 can be clamped in or extend into the gap of the pectinate muscle in LAA, i.e., a specific shape of the protrusion 131 may not be a limitation thereof.

In this embodiment, the protrusion 131 has a spherical shape, which ensures that the protrusion 131 does not cause damage to the pectinate muscle due to a tip when the protrusion 131 is clamped in or extends into the gap of the pectinate muscle in LAA. The anchoring member 13 reduces and avoids complications such as pericardial effusion when the medical device 100 is fixed in an occluded position, because it does not puncture or abrade the LAA wall.

Since the protrusion 131 actually resists the pectinate muscle in LAA, the protrusion 131 may be sized to meet certain conditions. Specifically, a maximum size (diameter) of the protrusion 131 is between 0.1 mm and 3 mm, preferably 1.5 mm-2.5 mm. It is to be noted that when the size of the protrusion 131 is less than 1.5 mm, the protrusion 131 may not be clamped in the tissue for a sufficient depth, resulting in a poor anchoring ability, and when the size of the protrusion 131 is greater than 2.5 mm, it is more difficult for the protrusion 131 to be clamped in the tissue, and sometimes impossible to be fully clamped in the tissue gap. In general, the protrusion 131 is sized at about 2 mm.

In addition, the protrusion 131 may be made of metal, polymer, or inorganic non-metal materials, or may be made of soft materials such as silicone and threads.

In this embodiment, each of the supporting rods 12 is provided with an anchoring member 13, i.e., the anchoring members 13 are uniformly distributed in a circumferential direction on the fixing part 10.

In another embodiment, a supporting rod 12 may also be provided with a plurality of anchoring members 13 to achieve a better fixation. Since there are gaps among anchoring members 13 on the same supporting rod 12 and the anchoring members 13 are clamped into different gaps of the pectinate muscle, no mutual interference occurs.

In another embodiment, the anchoring members 13 may be spaced apart on a plurality of supporting rods 12, i.e., not on each supporting rod 12 when the supporting force is sufficient, thereby reducing the number of anchoring members 13, further reducing the overall weight of the medical device 100 and the burden on the human body.

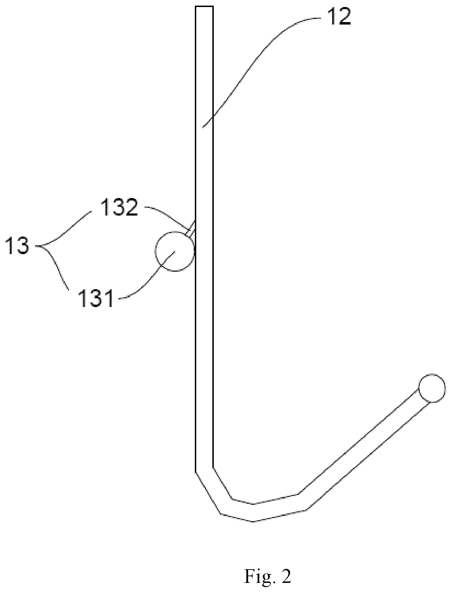

With regard to the specific structure of the anchoring member 13, please refer to FIG 2, which is a structural diagram of the anchoring member 13 of Embodiment 1 of the present invention. Assuming that a side close to the axis of the medical device 100 is a medial side and a side facing away from the axis is a lateral side, the protrusion 131 is positioned at the lateral side of the supporting rod 12, and the protrusion 131 extends obliquely outward with respect to the supporting rod 12. The length of the connecting member 132 may be set to 0.2 mm-4 mm. In a natural state, an opening angle of the connecting member 132 with respect to the axis of the medical device 100 is 0-90°. If the connecting member 132 is excessively long, the anchoring member 13 cannot fully enter the tissue gap, thus affecting the implantation diameter of the medical device 100 and the closeness to the human tissue, and further resulting in difficulty in sheathing the medical device 100. When the opening angle of the connecting member 132 (i.e., the opening angle of the anchoring member 13) is excessively small, the protrusion 131 is too close to the supporting rod 12 to be clamped into the tissue gap or abut against the tissue. When the opening angle of the connecting member 132 is excessively large, there will be difficulty in sheathing the medical device 100, and after the protrusion 131 is clamped in the tissue gap or abuts against the tissue, the force of the tissue acting on the anchoring member 13 follows the direction of the connecting member 132. In this case, the opening angle of the connecting member 132 is excessively large, resulting in that the component of the anchoring force of the anchoring member 13 along the axial direction becomes smaller, thus resulting in a decrease in the anchoring ability of the medical device 100. Therefore, in this embodiment, the length of the connecting member 132 is preferably 0.5-2 mm, and the opening angle is preferably 20°-60°.

Further, the connecting member 132 can be made of a hard material such as a hard metal rod or a hard polymer rod. In order to better adapt to pectinate muscle structures with different shapes and depths, the connecting member 132 can also be made of a flexible and soft material, such as a rod, thread, or elastic rope structure formed of a polymer, an inorganic material, or a flexible metal. The combination of the connecting member 132 with the protrusion 131 may be achieved by welding, heat-setting, hot melting, entanglement, etc.

For this embodiment, the plurality of anchoring members 13 are arranged such that the protrusions 131 thereof are positioned at the same horizontal plane or at the same distance or the same angle from the supporting rods 12 of the medical device 100. In order to facilitate movement into and out of the sheath, in other embodiments, the protrusions 131 of the plurality of anchoring members 13 may be positioned at different horizontal planes or at different distances and different angles from the supporting rods 12, so as to avoid stress concentration in the movement into and out of the sheath, i.e., at least one anchoring member 13 has a different opening angle and/or different horizontal position from adjacent anchoring members 13.

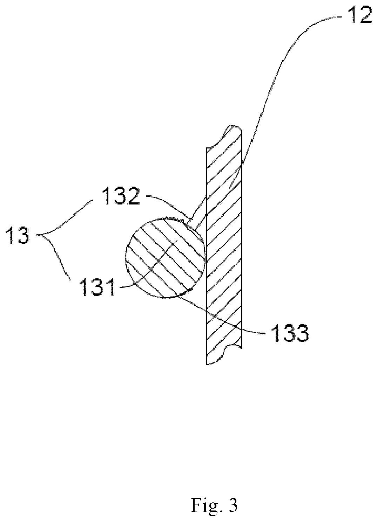

In another embodiment, referring to to FIG 3, which is a structural diagram of an anchoring member 13 in another embodiment of the present invention, in order to increase friction between the occluder and the atrial appendage and the stability of the anchor, a micro-barb structure 133 may be provided on the outer surface of the protrusion through a process such as adhesion, laser cutting, melting, or welding. When the protrusion 131 interact with the pectinate muscle, the micro-barb structure 133 can pierce into the pectinate muscle. In order to prevent complications such as pericardial effusion caused by the puncture of the atrial appendage wall by the micro-barb structure 133, the length of the micro-barb structure 133 may be less than 1 mm, and preferably 0.2-0.8 mm in this embodiment. In order to prevent complications such as embolism caused by the detachment of the micro-barb structure 133 in movement into and out of the sheath and implantation, the combination of the micro-barb structure 133 and the protrusion 131 is preferably achieved by an integral molding method such as a mold plus point contact melting, so as to ensure an excellent combination strength. The combination strength of the micro-barb structure 133 and the protrusion 131 shall be detected during the preparation of the product to ensure that the combination strength is greater than 10 N.

Further, the gap of the pectinate muscle and the atrial appendage wall have a concave shape, i.e., the bottom of the gap is the atrial appendage wall. In view of the interaction of the protrusion 131 with the atrial appendage wall and the pectinate muscle, in order to reduce the stimulation to the atrial appendage wall, the micro-barb structure 133 of the protrusion 131 can be localized, i.e., the micro-barb structure is provided at proximal and distal regions of the protrusion 131 contacting the atrial appendage wall, while the region at the lateral side of the protrusion 131 contacting the atrial appendage wall remains smooth. Specifically, at the contact surface of the protrusion 131 with the atrial appendage wall, the surface is smoothed by polishing, coating with a biocompatible and smooth coating, etc., without stimulating the atrial appendage wall. In addition, the micro-barb structure 133 may be provided at the contact surface of the protrusion 131 with the pectinate muscle to anchor the pectinate muscle and enhance the anchoring ability of the medical device 100.

It is to be noted that, in addition to avoiding the introduction of a sharp-tipped structure, the medical device 100 of this embodiment has further technical effects. Since the pectinate muscle generates contraction and relaxation along with the heart movement, the solution of anchoring with barbs (i.e., sharp-tipped ends) is likely to cause problems of penetrating excessively deeply into the LAA wall and even piercing through the LAA wall, and inevitably results in the expansion of the wound at the piercing site due to the movement, resulting in a decrease in the anchoring ability. For this embodiment, since the pectinate muscle at LAA is a crisscross mesh structure, the anchoring member of the medical device in this embodiment can be well clamped by the pectinate muscle when clamped into the gap of the pectinate muscle. Due to the contraction and relaxation of the pectinate muscle, the anchoring member which has not been fully clamped into the gap of the pectinate muscle in the implantation stage will be clamped further into the gap gradually with the movement of the pectinate muscle. An anchoring unit which has not been clamped into the gap in the implantation stage, if present, will also be clamped into the gap adaptively with the movement of the pectinate muscle, i.e., the medical device in this embodiment will further and adaptively increase the anchoring ability after implantation and maintain a good anchoring ability in coordination with contraction and relaxation of the pectinate muscle.

### Embodiment 2

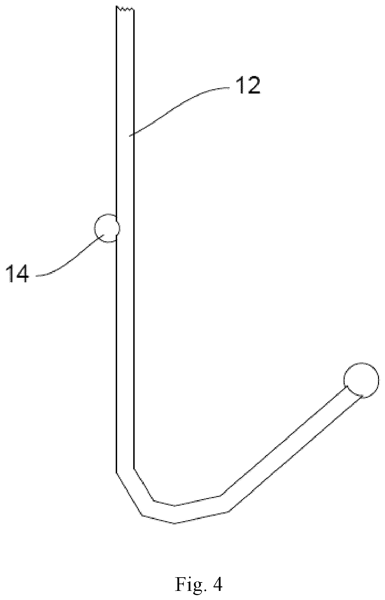

The medical device of Embodiment 2 differs from that of Embodiment 1 in that the anchoring unit of the anchoring member is directly connected to the supporting rod. With reference to FIG 4, which is a structural diagram of the anchoring member 14 of Embodiment 2 of the present invention, the anchoring member 14 (i.e., the anchoring unit of this embodiment) is arranged on the supporting rod 12 by welding, hot melting, entanglement, bonding, etc. The anchoring member 14 may have a protruding structure of a spherical, hemispherical, conical, polyhedral structure, etc., which is made of a material selected from metal, polymer, or inorganic non-metal materials, etc.

### Embodiment 3

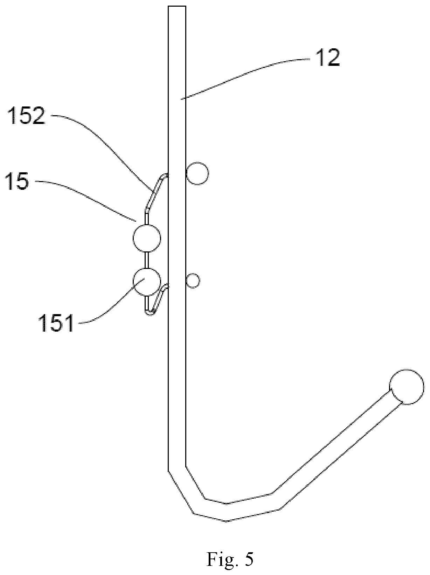

Embodiment 3 is configured based on Embodiment 1. The medical device of Embodiment 3 differs from that of Embodiment 1 in that the arrangement of the anchoring member of Embodiment 3 is different, with reference to FIG 5, which is a structural diagram of the anchoring member 15 of Embodiment 3. It is to be noted that the pectinate muscle has a complex structure with unevenly distributed gaps. Generally, when a protrusion 151 fails to enter a gap of the pectinate muscle, the protrusion 151 compresses the pectinate muscle, showing some anchoring ability, but the protrusion 151 has a better anchoring ability only when the protrusion 151 of the anchoring member 15 extends into or gets clamped in the gap. Therefore, in order to increase the probability that the protrusion 151 of the anchoring member 15 extends into or gets clamped in the gap, the anchoring member 15 is provided with a plurality of protrusions 151 on the same supporting rod 12.

The anchoring member 15 includes a flexible connecting member 152 to connect the plurality of protrusions 151 in series. Specifically, the flexible connecting member 152 is first connected to the supporting rod 12 by welding, hot melting, entanglement, bonding, etc. Alternatively, two holes are punched on the supporting rod 12, and then two ends of the flexible connecting member 152 pass through the two holes, separately. In general, both ends of the flexible connecting member 152 are fixed to the supporting rod 12 so as to prevent the protrusions 151 from swinging largely or shifting to a medial side of the supporting rod 12, resulting in losing the anchoring ability.

In addition, by limiting the relative positions of the plurality of protrusions 151 by the flexible connecting member 152, the stimulation to the atrial appendage wall by the protrusions upon contraction and relaxation of the atrial appendage can be effectively reduced, i.e., the protrusions 151 can follow the contraction and relaxation of the atrial appendage.

The flexible connecting member 152 is made of a thread or elastic rope with high toughness, elasticity, and strength. In this embodiment, the ends of the flexible connecting member 152 are knotted or melted to form knots having a diameter greater than a diameter of the holes so that the flexible connecting member 152 does not disengage from the supporting rod 12.

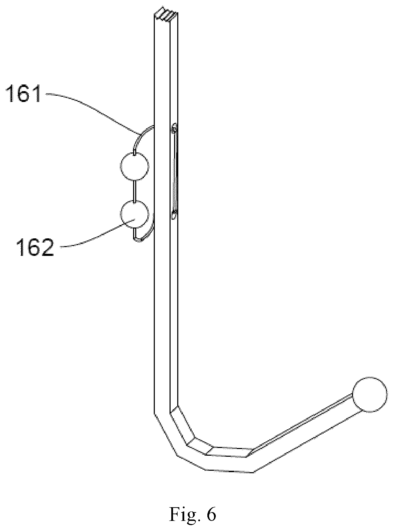

In another embodiment and as shown in FIG 6, in order to increase the range of motion of the flexible connecting member 162 and to avoid the knots loosening, causing the protrusions 161 to fall into the atrial appendage wall and other tissues, the ends of the flexible connecting member 162 may be connected to form a closed structure.

### Embodiment 4

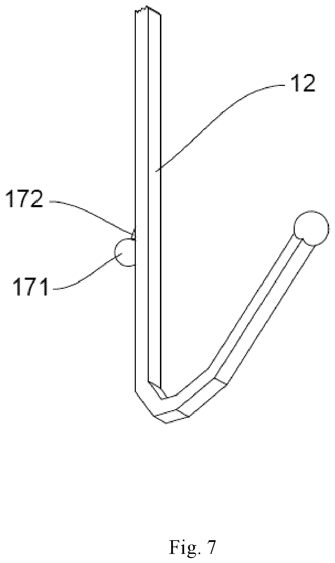

Embodiment 4 is configured based on Embodiment 1. The medical device of Embodiment 4 differs from that of Embodiment 1 in that the arrangement of the anchoring member of Embodiment 4 is different, with reference to FIG 7, where the protrusion 171 is connected to the supporting rod 12 via the connecting member 172.

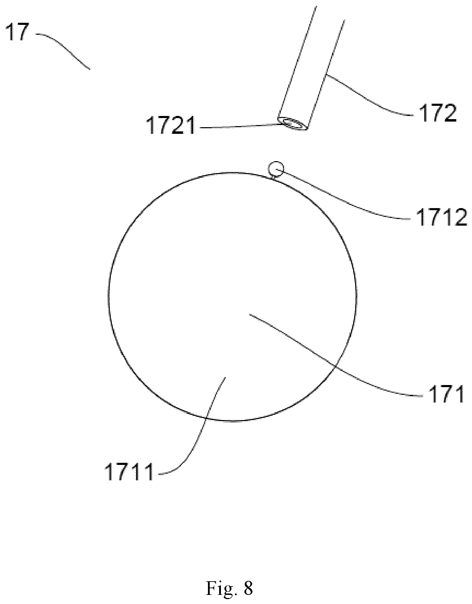

In order to further illustrate the combination method of the protrusion 171 with the connecting member 172, reference is further made to FIG 8, which is an exploded view of the anchoring member 17 of Embodiment 4. The anchoring member 17 includes a protrusion 171 and a connecting member 172, the protrusion 171 includes a first protrusion 1711 and a second protrusion 1712, and a receiving cavity 1721, which is configured to receive the second protrusion 1712, is arranged at a proximal end of the connecting member 172. The second protrusion 1712 is spherical and can rotate in multiple directions in the receiving cavity 1721 after being clamped in the receiving cavity 1721. The diameter of the second protrusion 1712 is greater than the diameter of the opening of the receiving cavity 1721, so that the second protrusion 1712 cannot disengage from the receiving cavity 1721 along the direction of the connecting member 172, but can freely rotate in the receiving cavity 1721.

The second protrusion 1712 may drive the first protrusion 1711 to rotate with respect to the connecting member 172 in multiple directions to reduce damage to the atrial appendage wall and the pectinate muscle by the end of the anchoring member during contraction and relaxation of the atrial appendage.

### Embodiment 5

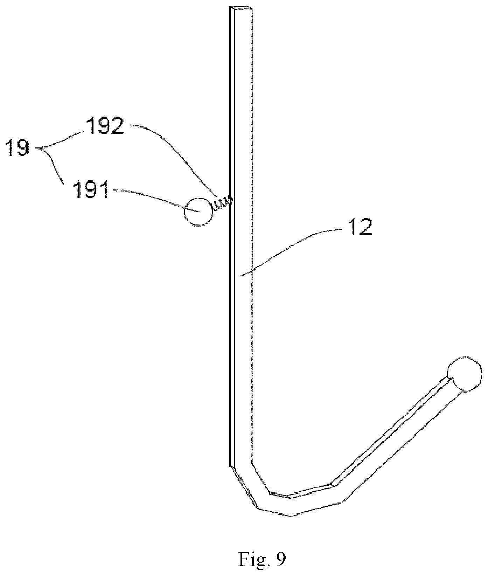

Embodiment 5 is configured based on Embodiment 1. The medical device of Embodiment 5 differs from that of Embodiment 1 in that the arrangement of the anchoring member of Embodiment 5 is different, with reference to FIG 9, which is a structural diagram of the anchoring member 19 of Embodiment 5 of the present invention. The anchoring member 19 includes a protrusion 191 and a connecting member 192 which connects the protrusion 191 to the supporting rod 12, where the connecting member 192 has a spring structure. Due to the properties of the spring, the connecting member 192 may drive the protrusion 191 to rotate in multiple directions, but at the same time apply a restoring force to the protrusion 191 in a restoring direction, so as to show a good anchoring ability throughout the contraction and relaxation of the atrial appendage.

In addition, since the atrial appendage contracts and relaxes as the heart beats, the protrusion 191 can adaptively shift along with the contraction and relaxation of the atrial appendage, so as not to damage or abrade the pectinate muscle and/or the atrial appendage wall due to the motion of the atrial appendage.

### Embodiment 6

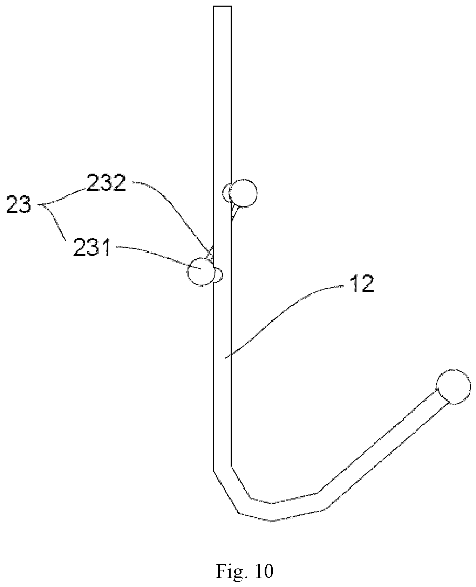

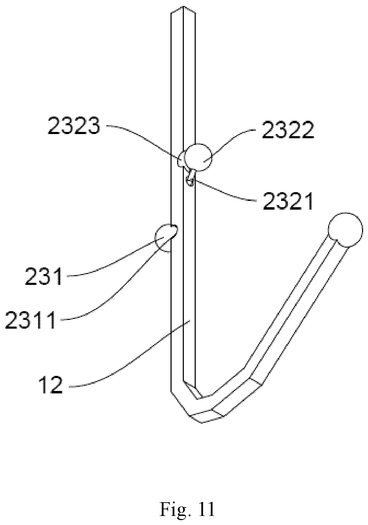

Embodiment 6 is configured based on Embodiment 1. The medical device of Embodiment 6 differs from that of Embodiment 1 in that the arrangement of the anchoring member of Embodiment 6 is different, with reference to FIG 10, which is a structural diagram of the anchoring member 23 of Embodiment 6 of the present invention, and FIG 11, which is a structural diagram of the anchoring member 23 of Embodiment 6 of the present invention form another perspective.

The anchoring member 23 includes a protrusion 231 and a connecting member 232 which connects the protrusion 231 to the supporting rod 12. The connecting member 232 includes a connecting rod 2321 and a connecting block 2322, where the connecting rod 2321 passes through a channel running through medial and lateral sides of the supporting rod 12 and connects to the connecting block 2322. The diameter of the connecting block 2322 is greater than the diameter of the channel where the connecting rod 2321 is positioned, so that the connecting block 2322 is always positioned at the medial side of the supporting rod 12, thereby further controlling the protrusion 231 to be positioned at the lateral side of the supporting rod 12. The connecting rod 2321 is made of a spring or a metal material with a strong restoring ability, such as a nickel-titanium wire.

Further, the supporting rod 12 is provided with a receiving recess 2311 that may at least partially receive the protrusion 231 and a receiving recess 2323 that may at least partially receive the connecting block 2322. When the medical device is integrally sheathed and transported in the sheath, the protrusion 231 and the connecting block 2322 are at least partially received in the receiving recess 2311 and the receiving recess 2323, respectively, so that the sheathing volume can be greatly reduced, thereby facilitating the integral sheathing and transportation of the medical device.

It is to be noted that the receiving recess 2311 and the receiving recess 2323 are not indispensablestructures. When the medical device is in a compressed state, the connecting rod 2321 can slide along the channel where it is positioned, and as the supporting rod 12 is compressed, the protrusion 231 positioned at the lateral side of the supporting rod 12 is also compressed toward the axial direction of the medical device, driving the connecting rod 2321 to slide along the channel for a distance toward the axial direction, thereby reducing the sheathing volume of the medical device.
In addition, it is to be further noted that the connecting block 2322 is not indispensable. The connecting rod 2321 may be directly connected to the supporting rod 12 by welding, bonding, and the like when the receiving recess 2311 is present, which also achieves the technical effect of reducing the sheathing volume of the medical device.

### Embodiment 7

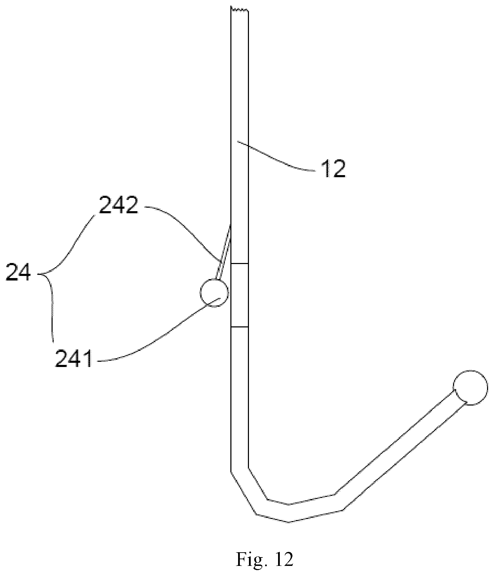

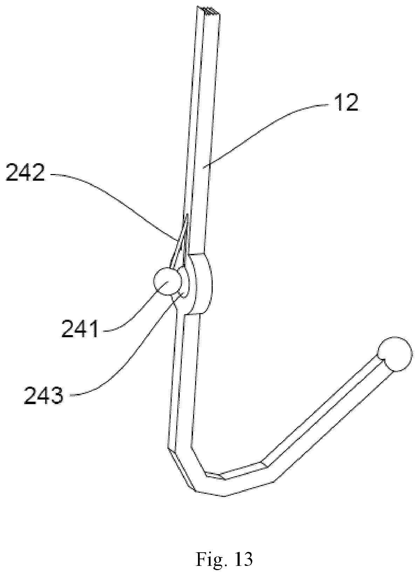

Embodiment 7 is configured based on Embodiment 1. The medical device of Embodiment 7 differs from that of Embodiment 1 in that the arrangement of the anchoring member of Embodiment 7 is different, with reference to FIG 12, which is a structural diagram of the anchoring member 24 of Embodiment 7 of the present invention, and FIG 13, which is a structural diagram of the anchoring member 24 of Embodiment 7 of the present invention form another perspective.

The anchoring member 24 includes a protrusion 241 and a connecting member 242 which connects the protrusion 241 to the supporting rod 12. It is to be noted that a through hole 243 is arranged on the supporting rod 12, where the diameter of the through hole 243 is greater than the diameter of the protrusion 241.

When the medical device is integrally sheathed and transported in the sheath, the protrusion 241 is pressed into the through hole 243 such that the protrusion 241 is at least partially received in the through hole 243, thereby greatly reducing the overall sheathing volume of the medical device, so as to facilitate sheathing and transportation of the medical device.

It is to be noted that the through hole 243 may be provided in other embodiments as a recess structure opening toward the protrusion 241, which also functions to at least partially receive the protrusion 241 when the medical device is in a compressed state. Therefore, both the through hole 243 and the recess structure are receiving cavities configured to receive or partially receive the protrusion 241, the receiving cavities receiving or partially receiving the protrusion 241 when the medical device is in a compressed state.

Furthermore, with regard to a receiving cavity, when the diameter of the receiving cavity is greater than or equal to the maximum diameter of the protrusion 241, the receiving cavity may theoretically receive all of the protrusion 241 (when the receiving cavity is a through hole structure or when the receiving cavity is a recess structure with a sufficient depth), which achieves an optimal technical effect of reducing the sheathing volume. However, when the diameter of the receiving cavity is smaller than the protrusion 241, the receiving cavity may partially receive the protrusion 241 as well, which may also achieve a certain technical effect of reducing the sheathing volume of the medical device. Therefore, the diameter of the receiving cavity may not be limited.

With the medical device of the above embodiments, the protrusions are clamped in gaps of the pectinate muscle after the medical device is released from the sheath, and due to the configuration of the protrusions, the protrusions do not damage the delicate pectinate muscle or pierce through the atrial appendage wall to cause pericardial effusion. The fixation of the medical device in an intended position by utilizing the gaps of the pectinate muscle ensures a good anchoring ability of the medical device while avoiding or substantially reducing damage to the intended implantation site, and therefore achieving a normal occluding function.

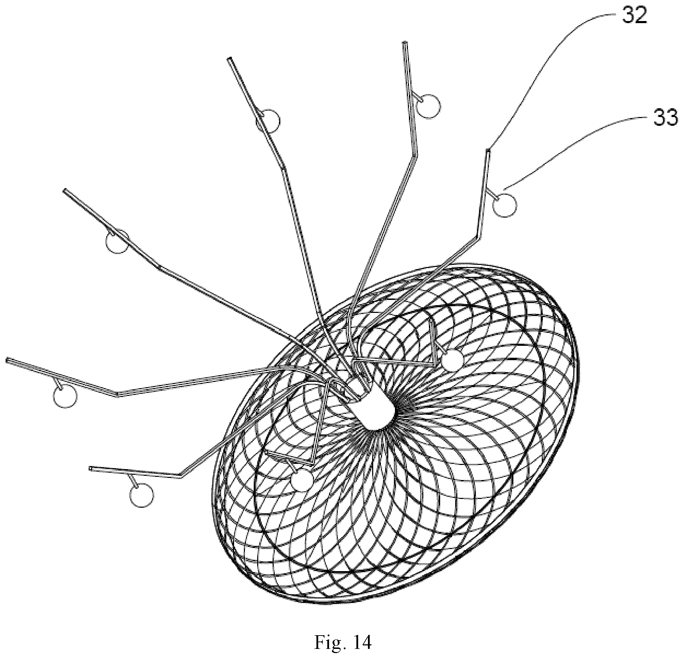

In addition to the improvements to the anchoring member in the embodiments described above, it is to be noted that the anchoring member is applicable to a variety of LAA occluders, with reference to FIGS. 14-17, which illustrate one state of application of the anchoring member to a first LAA occluder, a second LAA occluder, a third LAA occluder, and a fourth LAA occluder, separately.

With reference to FIG 14, which is a structural diagram showing application of the anchoring member 33 of the present invention to the first LAA occluder, when the anchoring member 33 is applied to the first LAA occluder, the anchoring member 33 is opened at an angle from the axis 2 of the medical device which is an included angle (acute angle) between the straight line where the anchoring member 33 is positioned and the axis.

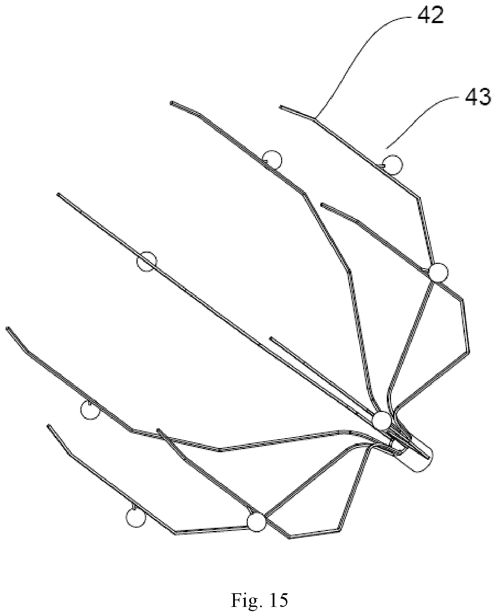

With reference to FIG 15, which is a structural diagram showing application of the anchoring member 43 of the present invention to the second LAA occluder, where the cover membrane structure is omitted in FIG 15, the cover membrane structure of the second LAA occluder is positioned at the end where a plurality of supporting rods 12 converge, i.e., the supporting rods 12 serve both a sealing function and a fixing function. In practice, the proximal end (i.e., the converging end) of the supporting rod 42 serves an occluding function for the sealing part, and the distal end serves a fixing function for the fixing part. The fixing part includes the supporting rod 42, and therefore, the second LAA occluder may be considered including the fixing part. Besides, the anchoring member 43 is positioned on a side of the fixing part close to the distal end. It will be appreciated that in other embodiments, the plurality of supporting rod 12 may be interconnected to form a grid-like sidewall, and are not limited to this embodiment where the plurality of supporting rods 12 are spaced apart from each other except for the end points. It will also be appreciated that in other embodiments, the plurality of anchoring members 43 may be interconnected to form an annular anchoring member surrounding the plurality of supporting rods 42.

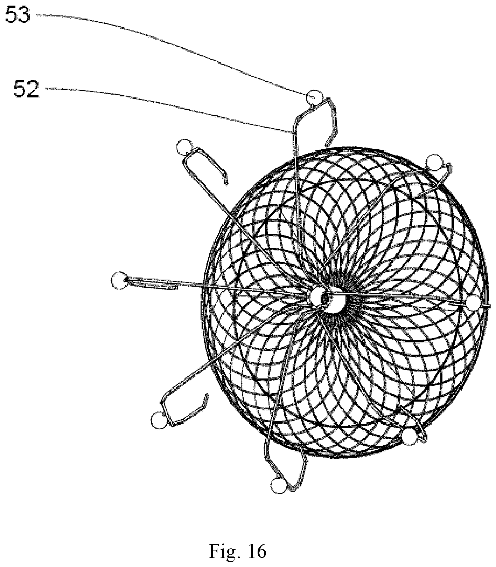

With reference to FIG 16, which is a structural diagram showing application of the anchoring member 53 of the present invention to the third LAA occluder, the anchoring member 53 is positioned on the supporting rod 52.

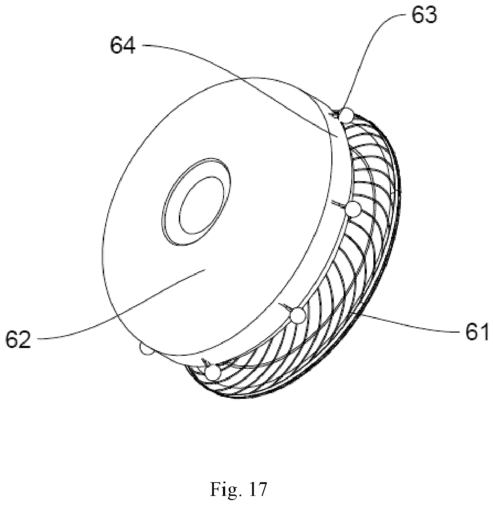

With reference to FIG 17, which is a structural diagram showing application of the anchoring member 63 of the present invention to the fourth LAA occluder, the fourth LAA occluder includes a sealing part 61 and a fixing part 62, where the fixing part 62 is provided with a cover membrane 64 through which the anchoring member 63 passes and extends toward a lateral side of the LAA occluder.

### Embodiment 8

The medical device of Embodiment 8 differs from those of Embodiments 1 to 7 in that the anchoring member of Embodiment 8 is provided on the cover membrane. It is to be noted that modifications and variations based on Embodiment 1 (i.e., Embodiments 1 to 7) may be equally applied to Embodiment 8 as well as other embodiments based on Embodiment 8 as long as the position of the anchoring member is set as in Embodiment 8.

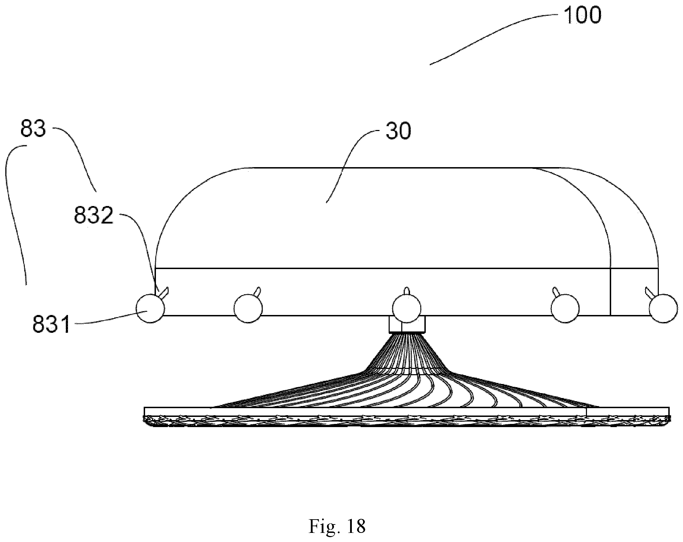

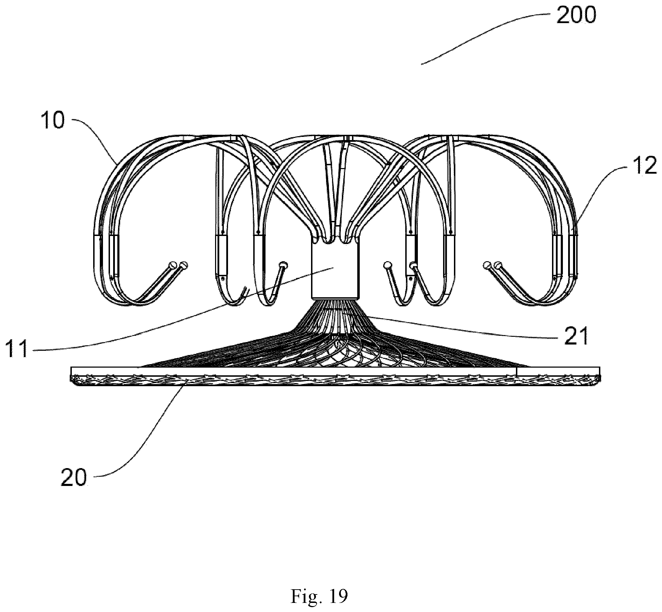

As shown in FIG 18, which is a structural diagram of a medical device of Embodiment 8 of the present invention, and FIG 19, which is a structural diagram of a main body of the medical device of Embodiment 8 of the present invention, the medical device includes a main body 200 including a fixing part 10 and a sealing part 20 connected to the fixing part 10, where a cover membrane 30 is covered outside the fixing part 10, and the sealing part 20 and the fixing part 10 are spaced apart along the axial direction of the medical device 100. The sealing part 20 is positioned at a proximal end of the medical device 100, and the fixing part 10 is positioned at a distal end of the medical device 100. The medical device 100 has a compressed state as housed within a sheath to facilitate delivery and a deployed state as shown in FIG 18 after extending from a distal end of the sheath and self-expanding for deployment. The shape of the medical device 100 after release into the LAA cavity is identical or substantially identical to that of FIG 18.

The sealing part 20 may be formed by weaving a plurality of woven wires into a mesh tube, and then closing and fixing ends of the woven wires by a plug 21 at a distal end of the sealing part 20 separately at two ends of the mesh tube. Then, the mesh tube is heat-set into a shape such as a disc shape, a cylindrical shape, or a plug shape, thereby obtaining a sealing part 20 for occluding the LAA ostium. At least one layer of film may be provided inside the sealing part 20 (not shown), the edge of which is fixed to the woven wires at the edge of the sealing part 20. The film serves to prevent blood flow from one side to the other side of the sealing part 20 so as to prevent blood flow between LAA and the left atrium.

The fixing part 10 includes a plug 11 of the fixing part and a plurality of supporting rods 12, and the plug 21 at the distal end of the sealing part is connected to the plug 11 of the fixing part via a connecting member 30. The supporting rods 12 of the fixing part 10 may be rods obtained by cutting a metal alloy tube or a polymer tube, or may be rods formed by weaving or winding woven wires.

As shown in FIG 19, proximal ends of the plurality of supporting rods 12 are connected to the plug 11 of the fixing part, and distal ends extend radially outward from a central end portion and turn toward the sealing part, thereby forming a support surface for contacting the LAA cavity wall and performing a support function.

The cover membrane 30 is provided with anchoring members 83. The material of the cover membrane 30 can be a polymer material, an inorganic material, or a metal material, and in general, the anchoring members 83 are arranged on the cover membrane 30 by melting, entanglement, bonding, etc.

In this embodiment, the anchoring member 83 includes an anchoring unit and a connecting unit, where the anchoring unit in this embodiment is preferably a protrusion 831, and the connecting unit is selected as a rod-shaped connecting member 832, where one end of the connecting member 832 is connected to the cover membrane 30, and the other end is connected to the protrusion 831. The protrusion 831 may have a spherical, hemispherical, cylindrical, conical, polyhedral structure, etc., as long as the protrusion 831 can be clamped in or extend into the gap of the pectinate muscle in LAA, i.e., a specific shape of the protrusion 831 may not be a limitation thereof.

In this embodiment, the protrusion 831 has a spherical shape, which ensures that the protrusion 831 does not cause damage to the pectinate muscle due to a tip when the protrusion 831 is clamped in or extends into the gap of the pectinate muscle in LAA. The anchoring member 83 reduces and avoids complications such as pericardial effusion when the medical device 100 is fixed in an occluded position, because it does not puncture or abrade the LAA wall.

Since the protrusion 831 actually resists the pectinate muscle in LAA, the protrusion 831 may be sized to meet certain conditions. Specifically, a maximum size (diameter) of the protrusion 831 is between 0.1 mm and 3 mm, preferably 1.5 mm-2.5 mm. It is to be noted that when the size of the protrusion 831 is less than 1.5 mm, the protrusion 831 may not be clamped in the tissue for a sufficient depth, resulting in a poor anchoring ability, and when the size of the protrusion 831 is greater than 2.5 mm, it is more difficult for the protrusion 831 to be clamped in the tissue, and sometimes impossible to be fully clamped in the tissue gap. In general, the protrusion 831 is sized at about 2 mm.

In addition, the protrusion 831 may be made of metal, polymer, or inorganic non-metal materials, or may be made of soft materials such as silicone and threads.

In this embodiment, the supporting rods 12 divide the cover membrane 30 into a plurality of regions, and the plurality of protrusions 831 are separately located in middle positions of the regions, i.e., the anchoring members 83 are uniformly distributed in a circumferential direction on the surface of the cover membrane 30.

In another embodiment, the cover membrane 30 is provided with a plurality of anchoring members 83 not only in the circumferential direction, but also in the axial direction, i.e., more than one protrusion 831 in each region, so as to obtain a better fixation of the medical device 100. Since there are gaps among anchoring members 83 and the anchoring members 83 are clamped into different gaps of the pectinate muscle, no mutual interference occurs.

In another embodiment, the supporting rods 12 divide the cover membrane 30 into a plurality of regions, and the anchoring members 83 are spaced apart on the plurality of regions of the cover membrane 30, i.e., reducing the number of anchoring members 83 when the supporting force is sufficient, thereby reducing the overall weight of the medical device 100 and the burden on the human body.

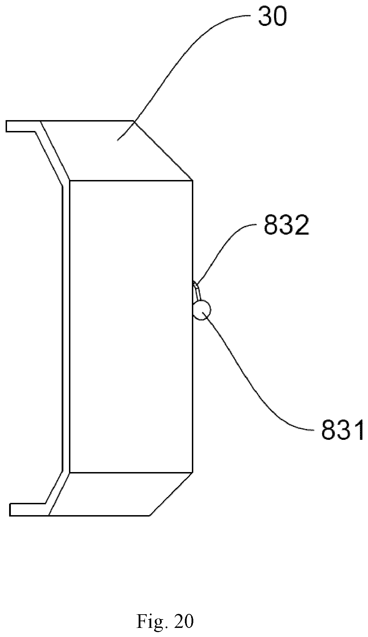

With regard to the specific structure of the anchoring member 83, please refer to FIG 20, which is a structural diagram of the anchoring member 83 of Embodiment 8 of the present invention. Assuming that a side close to the axis of the medical device 100 is a medial side and a side facing away from the axis is a lateral side, the protrusion 831 is positioned at the lateral side of the cover membrane 30, and the protrusion 831 extends obliquely outward with respect to the cover membrane 30. The length of the connecting member 832 may be set to 0.2 mm-4 mm. In a natural state, an opening angle of the connecting member 832 with respect to the axis of the medical device 100 is 0-90°. If the connecting member 832 is excessively long, the anchoring member 83 cannot fully enter the tissue gap, thus affecting the implantation diameter of the medical device 100 and the closeness to the human tissue, and further resulting in difficulty in sheathing the medical device 100. When the opening angle of the connecting member 832 (i.e., the opening angle of the anchoring member 83) is excessively small, the protrusion 831 is too close to the cover membrane 30 to be clamped into the tissue gap or abut against the tissue. When the opening angle of the connecting member 832 is excessively large, there will be difficulty in sheathing the medical device 100, and after the protrusion 831 is clamped in the tissue gap or abuts against the tissue, the force of the tissue acting on the anchoring member 83 follows the direction of the connecting member 832. In this case, the opening angle of the connecting member 832 is excessively large, resulting in that the component of the anchoring force of the anchoring member 83 along the axial direction becomes smaller, thus resulting in a decrease in the anchoring ability of the medical device 100. Therefore, in this embodiment, the length of the connecting member 832 is preferably 0.5-2 mm, and the opening angle is preferably 20°-60°.

Further, the connecting member 832 can be made of a hard material such as a hard metal rod or a hard polymer rod. In order to better adapt to pectinate muscle structures with different shapes and depths, the connecting member 832 can also be made of a flexible and soft material, such as a rod, thread, or elastic rope structure formed of a polymer, an inorganic material, or a flexible metal. The combination of the connecting member 832 with the protrusion 831 may be achieved by welding, heat-setting, hot melting, entanglement, etc.

In another embodiment, the anchoring member 83 includes a protrusion 831 and a connecting member 832 which connects the protrusion 831 to the cover membrane 30, where the connecting member 122 is a resilient member. Due to the properties of the resilient member, the connecting member 122 may drive the protrusion 121 to rotate in multiple directions, but at the same time apply a restoring force to the protrusion 121 in a restoring direction, so as to show a good anchoring ability throughout the contraction and relaxation of the atrial appendage.

In addition, since the atrial appendage contracts and relaxes as the heart beats, the protrusion 121 can adaptively shift along with the contraction and relaxation of the atrial appendage, so as not to damage or abrade the pectinate muscle and/or the atrial appendage wall due to the motion of the atrial appendage.

For this embodiment, the plurality of anchoring members 83 are arranged such that the protrusions 831 thereof are positioned at the same horizontal plane or at the same distance or the same angle from the cover membrane 30 of the medical device 100. In order to facilitate movement into and out of the sheath, in other embodiments, the protrusions 831 of the plurality of anchoring members 83 may be positioned at different horizontal planes or at different distances and different angles from the axis of the medical device 100, so as to avoid stress concentration in the movement into and out of the sheath, i.e., at least one anchoring member 83 has a different opening angle and/or different horizontal position from adjacent anchoring members 83.

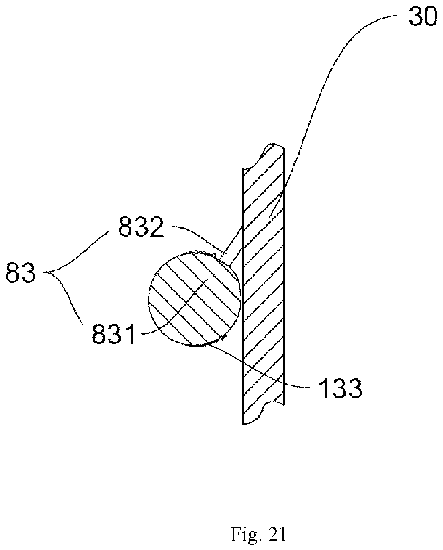

In another embodiment and with reference to FIG 21, which is a structural diagram of an anchoring member 83 in another embodiment of the present invention, in order to increase friction between the occluder and the atrial appendage and the stability of the anchor, a micro-barb structure 833 may be provided on the outer surface of the protrusion through a process such as adhesion, laser cutting, melting, or welding. When the protrusion 831 interact with the pectinate muscle, the micro-barb structure 833 can pierce into the pectinate muscle. In order to prevent complications such as pericardial effusion caused by the puncture of the atrial appendage wall by the micro-barb structure 833, the length of the micro-barb structure 833 may be less than 1 mm, and preferably 0.2-0.8 mm in this embodiment. In order to prevent complications such as embolism caused by the detachment of the micro-barb structure 833 in movement into and out of the sheath and implantation, the combination of the micro-barb structure 833 and the protrusion 831 is preferably achieved by an integral molding method such as a mold plus point contact melting, so as to ensure an excellent combination strength. The combination strength of the micro-barb structure 833 and the protrusion 831 shall be detected during the preparation of the product to ensure that the combination strength is greater than 10 N.

Further, the gap of the pectinate muscle and the atrial appendage wall have a concave shape, i.e., the bottom of the gap is the atrial appendage wall. In view of the interaction of the protrusion 831 with the atrial appendage wall and the pectinate muscle, in order to reduce the stimulation to the atrial appendage wall, the micro-barb structure 833 of the protrusion 831 can be localized, i.e., the micro-barb structure is provided at proximal and distal regions of the protrusion 831 contacting the atrial appendage wall, while the region at the lateral side of the protrusion 831 contacting the atrial appendage wall remains smooth. Specifically, at the contact surface of the protrusion 831 with the atrial appendage wall, the surface is smoothed by polishing, coating with a biocompatible and smooth coating, etc., without stimulating the atrial appendage wall. In addition, the micro-barb structure 833 may be provided at the contact surface of the protrusion 831 with the pectinate muscle to anchor the pectinate muscle and enhance the anchoring ability of the medical device 100.

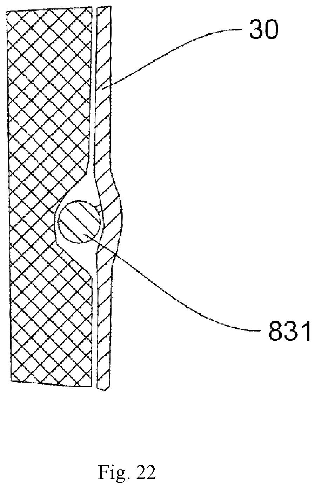

With reference to FIG 22, which is a working diagram of the anchoring member 83 after implantation of the medical device 100 of Embodiment 8 of the present invention, it is to be noted that the cover membrane 30 has a flexible structure. After implantation of the medical device 100, the protrusion 831 is clamped in the pectinate muscle, and the protrusion 831 serves an anchoring function regardless of whether a free end of the protrusion 831 contacts the LAA wall. The protrusion 831 is subjected to the stress of the pectinate muscle (or to a combined stress of the pectinate muscle and the LAA wall), and the stress is transmitted along the connecting member 832 to a contact position of the cover membrane 30 with the connecting member 832, so as to drive the contact position of the cover membrane 30 with the connecting member 832 to move toward the axial direction of the medical device 100, thus forming a depressed region at the contact position of the connecting member 832 with the cover membrane 30. Meanwhile, in order to compensate for the stress at the contact position of the connecting member 832 with the cover membrane 30 (toward the axial direction of the medical device 100) at an edge portion of the depressed region, the stress in the edge portion faces away from the axial direction of the medical device 100, i.e., close to the LAA wall. Therefore, the edge portion of the depressed region abuts closely against the inner wall of the LAA structure (including the pectinate muscle), resulting in a good auxiliary sealing effect of the cover membrane 30.

Further, the connecting position of the connection member 832 with the cover membrane 30 is positioned at a center of the depressed region. If stitching is used, small holes will be formed on the surface of the cover membrane which may result in damage to the cover membrane 30. A portion of the cover membrane 30 with small holes is positioned at the center of the depressed region, and the edge of the depressed region ensures the auxiliary sealing effect of the cover membrane 30.

It is to be noted that if the connecting member 832 has a flexible rope structure, the protrusion 831 will directly press against the cover membrane 30 to form a depressed structure. On this basis, the protrusion 831 is subjected to the stress of the pectinate muscle (or to a combined stress of the pectinate muscle and the LAA wall), and the stress is transmitted along the protrusion 831 to the surface of the cover membrane 30 to form a depressed structure, which can also achieve the same technical effect.

It is to be noted that, in addition to avoiding the introduction of a sharp-tipped structure, the medical device 100 of this embodiment has further technical effects. Since the pectinate muscle generates contraction and relaxation along with the heart movement, the solution of anchoring with barbs (i.e., sharp-tipped ends) is likely to cause problems of penetrating excessively deeply into the LAA wall and even piercing through the LAA wall, and inevitably results in the expansion of the wound at the piercing site due to the movement, resulting in a decrease in the anchoring ability. For this embodiment, since the pectinate muscle at LAA is a crisscross mesh structure, the anchoring member of the medical device in this embodiment can be well clamped by the pectinate muscle when clamped into the gap of the pectinate muscle. Due to the contraction and relaxation of the pectinate muscle, the anchoring member which has not been fully clamped into the gap of the pectinate muscle in the implantation stage will be clamped further into the gap gradually with the movement of the pectinate muscle. An anchoring unit which has not been clamped into the gap in the implantation stage, if present, will also be clamped into the gap adaptively with the movement of the pectinate muscle, i.e., the medical device in this embodiment will further and adaptively increase the anchoring ability after implantation and maintain a good anchoring ability in coordination with contraction and relaxation of the pectinate muscle.

### Embodiment 9

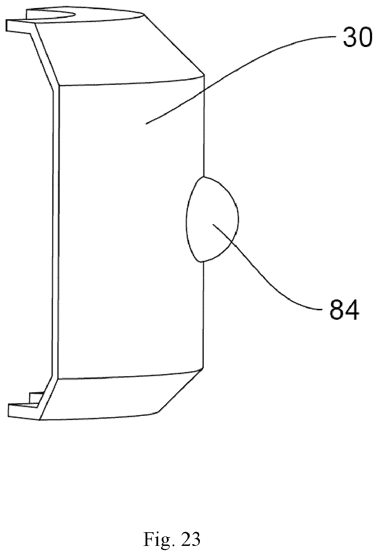

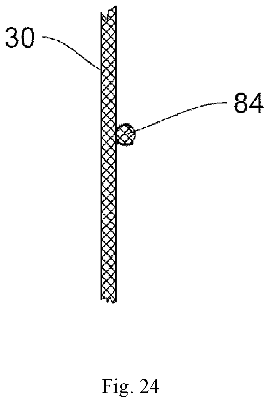

The medical device of Embodiment 9 differs from that of Embodiment 8 in that the anchoring unit of the anchoring member is directly positioned on the surface of the cover membrane. With particular reference to FIG 23, which is a structural diagram of the anchoring member 84 of Embodiment 9 of the present invention, and FIG 24, which is a cross-sectional diagram of the anchoring member 84 of Embodiment 9 of the present invention, the anchoring member 84 (i.e., the anchoring unit of this embodiment) is arranged on the cover membrane 30 by hot melting, entanglement, bonding, etc. In this embodiment, the hot melting is used, and the anchoring member 84 may have a protruding structure of a spherical, hemispherical, conical, polyhedral structure, etc., which is made of a material selected from metal, polymer, or inorganic non-metal materials, etc.

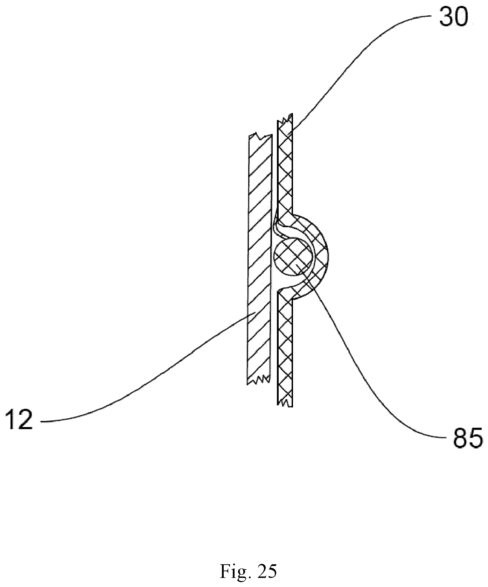

In another embodiment and with reference to FIG 25, which is a structural diagram of the anchoring member 85 of the medical device in another embodiment of the present invention, the anchoring member 85 is positioned between the supporting rod 12 and the cover membrane 30, and the cover membrane 30 abuts closely against the surface of the anchoring member 85 to form a protrusion structure, thereby performing an anchoring function.

### Embodiment 10

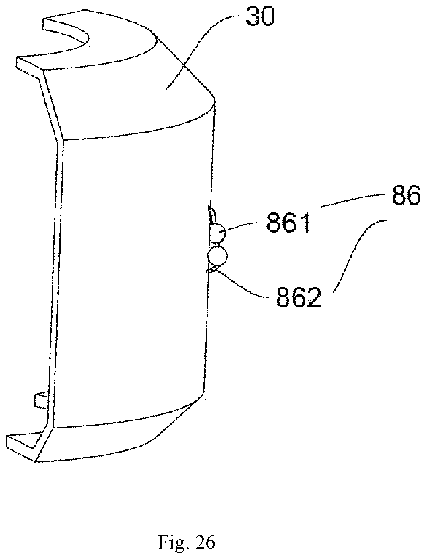

Embodiment 10 is configured based on Embodiment 8. The medical device of Embodiment 10 differs from that of Embodiment 8 in that the arrangement of the connecting unit and the anchoring unit of the anchoring member of Embodiment 10 is different, with reference to FIG 26, which is a structural diagram of the anchoring member 86 of Embodiment 10. It is to be noted that the pectinate muscle has a complex structure with unevenly distributed gaps. Generally, when a protrusion 861 fails to enter a gap of the pectinate muscle, the protrusion 861 compresses the pectinate muscle, showing some anchoring ability, but the protrusion 861 has a better anchoring ability only when the protrusion 861 of the anchoring member 86 extends into or gets clamped in the gap. Therefore, in order to increase the probability that the protrusions 861 of the anchoring member 86 extends into or gets clamped in the gap, a single anchoring member 86 includes a plurality of protrusions 861.

The anchoring member 86 includes a flexible connecting member 862 to connect the plurality of protrusions 861 in series. Specifically, the flexible connecting member 862 is first connected to the supporting rod 12 by welding, hot melting, entanglement, bonding, etc. Alternatively, two holes are punched on the cover membrane 30, and then two ends of the flexible connecting member 862 pass through the two holes, separately. In general, both ends of the flexible connecting member 862 are fixed to the cover membrane 30 so as to prevent the protrusions 861 from swinging largely which my result in losing the anchoring ability. Further, the two ends of the flexible connecting member 862 may pass through holes on the cover membrane 30 and joined together inside the cover membrane 30. Alternatively, one end of the flexible connecting member 862 may pass through a hole on the cover membrane 30 and become knotted so that the flexible connecting member 862 is movably fixed to the cover membrane 30.

In addition, by limiting the relative positions of the plurality of protrusions 861 by the flexible connecting member 862, the stimulation to the atrial appendage wall by the protrusions upon contraction and relaxation of the atrial appendage can be effectively reduced, i.e., the protrusions 861 can follow the contraction and relaxation of the atrial appendage.

The flexible connecting member 862 is made of a thread or elastic rope with high toughness, elasticity, and strength. In this embodiment, the ends of the flexible connecting member 862 are knotted or melted to form knots having a diameter greater than a diameter of the holes so that the flexible connecting member 862 does not disengage from the supporting rod 12.

In another embodiment, in order to increase the range of motion of the flexible connecting member 862 and to avoid the knots loosening, causing the protrusions 861 to fall into the atrial appendage wall and other tissues, the ends of the flexible connecting member 862 may be connected to form a closed structure.

### Embodiment 8

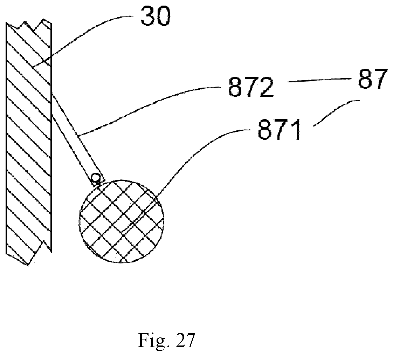

Embodiment 8 is configured based on Embodiment 8. The medical device of Embodiment 11 differs from that of Embodiment 8 in that the arrangement of the connecting unit and the anchoring unit of the anchoring member of Embodiment 11 is different, with reference to FIG 27, where the protrusion 871 is connected to the supporting rod 12 via the connecting member 872.

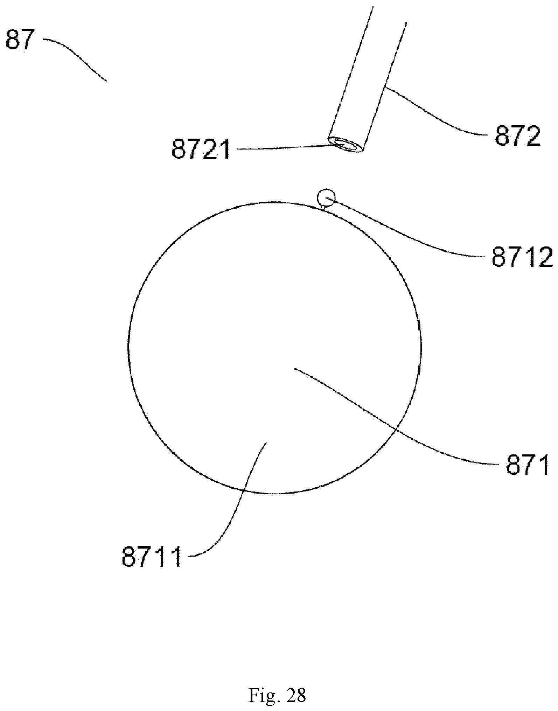

In order to further illustrate the combination method of the protrusion 871 with the connecting member 872, reference is further made to FIG 28, which is an exploded view of the anchoring member 87 of Embodiment 11. The anchoring member 87 includes a protrusion 871 and a connecting member 872, the protrusion 871 includes a first protrusion 8711 and a second protrusion 8712, and a receiving cavity 8721, which is configured to receive the second protrusion 8712, is arranged at a proximal end of the connecting member 872. The second protrusion 8712 is spherical and can rotate in multiple directions in the receiving cavity 8721 after being clamped in the receiving cavity 8721. The diameter of the second protrusion 8712 is greater than the diameter of the opening of the receiving cavity 8721, so that the second protrusion 8712 cannot disengage from the receiving cavity 8721 along the direction of the connecting member 872, but can freely rotate in the receiving cavity 8721.

The second protrusion 8712 may drive the first protrusion 8711 to rotate with respect to the connecting member 872 in multiple directions to reduce damage to the atrial appendage wall and the pectinate muscle by the end of the anchoring member during contraction and relaxation of the atrial appendage.

### Embodiment 12

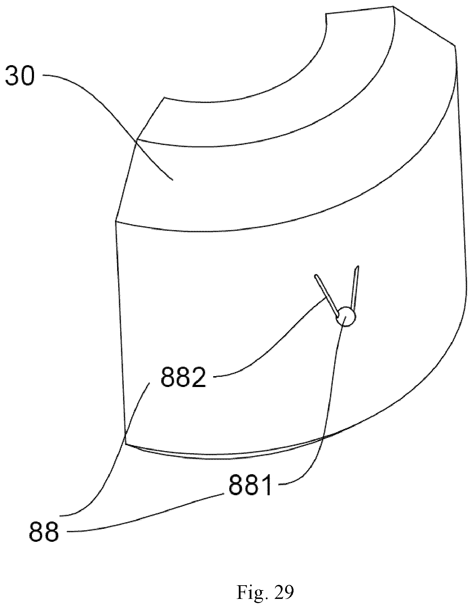

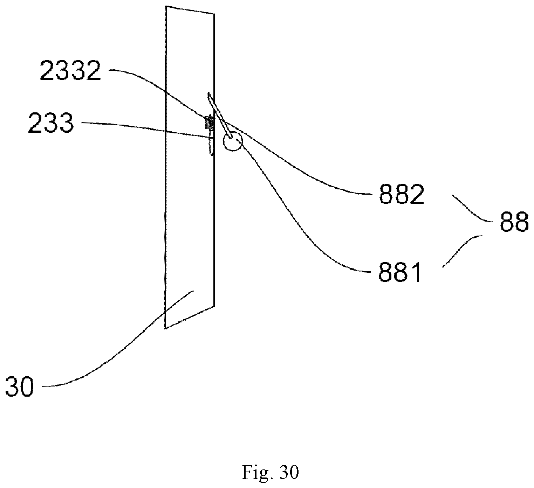

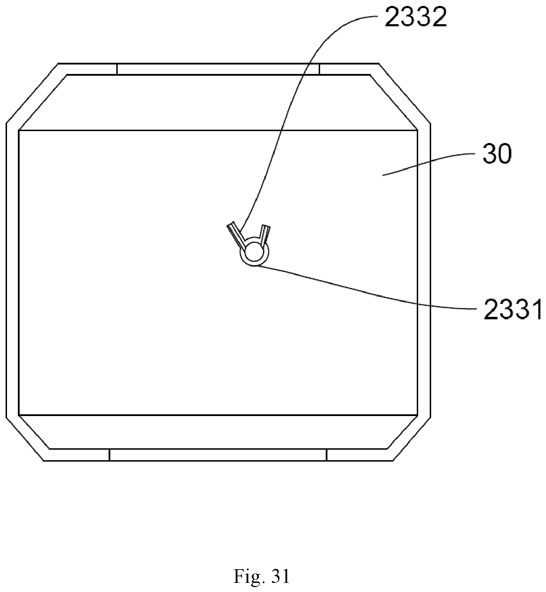

Embodiment 12 is configured based on Embodiment 8. The medical device of Embodiment 12 differs from that of Embodiment 8 in that the arrangement of the connecting unit and the anchoring unit of the anchoring member of Embodiment 12 is different, with reference to FIG 29, which is a structural diagram of the anchoring member 88 of Embodiment 13 of the present invention from a first perspective, FIG 30, which is a structural diagram of the anchoring member 88 of Embodiment 13 of the present invention from a second perspective, and FIG 31, which is a structural diagram of the anchoring member 88 of Embodiment 13 of the present invention from a third perspective.

The anchoring member 88 includes a protrusion 881 and a connecting member 882 configured to fix the protrusion 881 to the cover membrane 30. The connecting member 882 includes a first connecting rod 8821 and a second connecting rod 8822. In this embodiment, the connecting member 882 is a resilient member and supports the protrusion 881 in a natural state.

The surface of the cover membrane 30 is provided with a receiving recess 233, and the receiving recess 233 includes a first recess 2331 corresponding to the protrusion 881 and a second recess 2332 corresponding to the connecting member 882. It is to be noted that the receiving recess 233 may be arranged as a through recess running through the cover membrane 30.

When the medical device is integrally sheathed and transported in the sheath, the protrusion 881 is pressed into the receiving recess 233 such that the protrusion 881 is at least partially received in the receiving recess 233, thereby greatly reducing the overall sheathing volume of the medical device, so as to facilitate sheathing and transportation of the medical device. Most importantly, since the receiving recess 233 partially receives the protrusion 881, which actually limits the protrusion 881, the protrusion 881 is prevented from entangling with other structures of the medical device.

Furthermore, with regard to the receiving recess 233, when the diameter of the receiving recess 233 is greater than or equal to the maximum diameter of the protrusion 881, the receiving recess 233 may theoretically receive all of the protrusion 881 (when the receiving cavity is a through hole structure or when the receiving cavity is a recess structure with a sufficient depth), which achieves an optimal technical effect of reducing the sheathing volume. However, when the diameter of the receiving recess 233 is smaller than the protrusion 881, the receiving recess 233 may partially receive the protrusion 881 as well, which may also achieve a certain technical effect of reducing the sheathing volume of the medical device. Therefore, the diameter of the receiving cavity may not be limited.

### Embodiment 13

The medical device of Embodiment 13 differs from that of Embodiment 1 in that the fixing part of Embodiment 13 has a mesh structure. It is to be noted that modifications and variations based on Embodiment 1 may be equally applied to Embodiment 13 as well as other embodiments based on Embodiment 13 as long as the fixing part is arranged as in Embodiment 13.

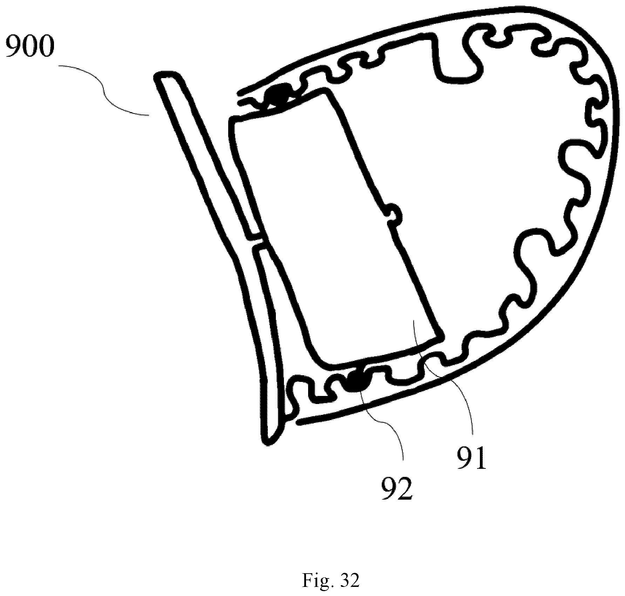

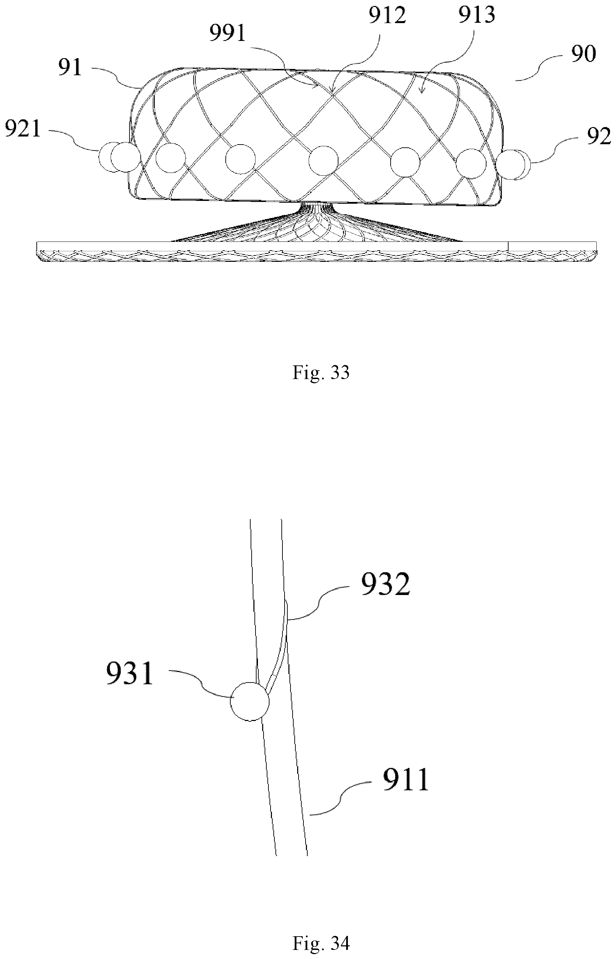

With reference to FIG 32, which is a schematic diagram showing an implantation state of the medical device of Embodiment 13 of the present invention, and FIG 33, which is a structural diagram of the medical device of Embodiment 13 of the present invention, the fixing part 90 includes a mesh support structure 91, which may generally assume a cylindrical shape, an umbrella shape, a plug shape, etc. The mesh support structure 91 includes a plurality of supporting wires 911 that converge to form a mesh 913. "One" of the supporting wires 911 as defined in the present invention refers to a division of supporting wires 911 of the mesh support structure 91 into a plurality with the intersection 912 (or crossing point) of the supporting wires 911 as a virtual division point. For some mesh support structures 11 that are actually formed by weaving a single continuous supporting wire 911, a plurality of supporting wires 911 are included as well with reference to the definition of the present invention.

The anchoring members 92 are mounted on the surface of the mesh support structure 91, and each anchoring member 92 includes an anchoring unit 921 which, in this embodiment, is a protrusion that may have a spherical, hemispherical, drop-shaped, cylindrical, conical, polyhedral structure, etc. It is to be noted that the protrusion is not the only manifestation of the anchoring unit 921, and the anchoring unit 921 may be configured in a variety of structures, such as annular, umbrella-shaped, disc-shaped, and radial, as long as it is ensured that the anchoring unit 921 can be adaptively clamped in or extend into the gap in the human tissue or abut against the human tissue. The anchoring unit 921 may be made of a material selected from one or more of metal, polymer, or inorganic non-metal materials, for example, hard materials such as nickel-titanium alloy, ceramics, PTFE, and PET, and soft materials such as silicone and threads.

In this embodiment, the mesh support structure 91 may be woven from a plurality of supporting wires 911, or may be obtained by cutting a tube member (e.g., a shape memory alloy tube, or a polymer tube), and is preferably woven from at least one metal wire in this embodiment. The supporting wire 911 may have a single wire structure or a plurality of wires that are helically wound. The material of the supporting wire 911 may be selected from one or more of a metal material (e.g., nickel-titanium), a polymer material, and an inorganic non-metal material. The present invention does limit the material and structure of the mesh support structure 91 as long as the mesh support structure 91 has a certain radial supporting force. A section of a single supporting wire 911 has the same structural features as the supporting rods in Embodiments 1-7. Therefore, this embodiment can directly refer to the combination method of the anchoring unit (i.e., the protrusion) with the supporting rod in Embodiments 1-7 for that in this embodiment.

### Embodiment 14

With reference to FIG. 34, the medical device of this embodiment is substantially identical to that of Embodiment 13, and the identical parts will not be described herein. The main difference lies in that the connecting unit 921 is connected to the supporting wire 911 by entanglement.

In this embodiment, the main body of the connecting unit 932 may be made of a hard metal, a hard polymer material, etc., or may be made of a flexible and soft material, such as a rod, wire, thread, or elastic rope structure formed by a soft polymer material, an inorganic material, or a flexible metal, etc.

The connecting unit 932 is wound around the supporting wire 911 and fixedly connected to the supporting wire 911, or the connecting unit 932 is slidable with respect to the supporting wire 911 along a length direction of the supporting wire 911. In this embodiment, the connecting unit 932 is wound around the supporting wire 911 for one turn, and in other embodiments, the connecting unit 932 may be wound around the supporting wire 911 for a plurality of turns.

In order to enhance the firmness of the connection between the connecting unit 932 and the supporting wire 911 when the connecting unit 932 is fixedly connected to the supporting wire 911, the connecting unit 932 and the supporting wire 911 may be secondarily fixed by welding, hot melting, bonding, etc.

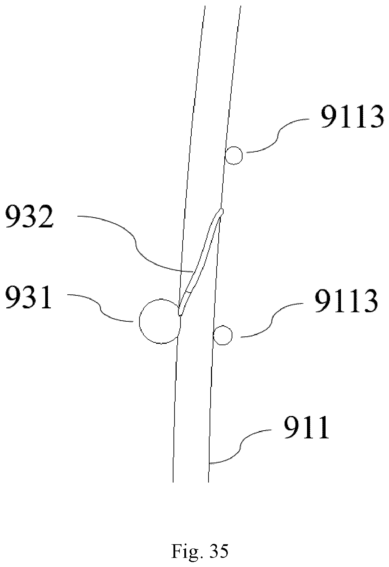

When the connecting unit 932 is slidable with respect to the supporting wire 911 along the length direction of the supporting wire 911, a limit unit may be provided on the supporting wire 911 to prevent the connecting unit 932 from sliding too much on the supporting wire 911. With reference FIG 35, for example, two third anchoring units 9113 may be provided on a medial side of the supporting wire 911 (i.e., a side of the supporting wire 911 close to the central axis of the medical device when the medical device is in a deployed state), and the two third anchoring units 9113 are separately arranged at proximal and distal sides of the connecting unit 932. In this embodiment, each third anchoring unit 9113 has a spherical shape. In other embodiments, each third anchoring unit 9113 may be rod-shaped and extend toward a medial side of the mesh support structure 11 when the medical device is in the deployed state, and may be pressed onto the supporting wire 911 or into a pre-set receiving cavity on the supporting wire 911 under a radial force, and resume the state extending toward the medial side of the mesh support structure 11 when the radial force is removed. This configuration is advantageous in that the radially compressed size of the medical device can be further reduced.

In other embodiments, a tapered region (not shown) having a smaller wire diameter than other regions of the supporting wire 911 may be provided on the supporting wire 911 as a limit unit, so as to prevent the connecting unit 932 from sliding to other regions of the supporting wire 911 with greater wire diameters when the connecting unit 932 is wound around the tapered region.

For a connecting unit 932 made of a hard material, when it is fixedly connected to the supporting wire 911, a better support function may be performed when the anchoring unit contacts with the atrial appendage wall 220. While the connecting unit 932 is slidable with respect to the supporting wire 911 along the length direction of the supporting wire 911, LAA contracts and relaxes as the heart beats, and the connecting unit 932 can adaptively shift along with the contraction and relaxation of the atrial appendage, so as not to damage or abrade the pectinate muscle 210 and/or the atrial appendage wall 220 due to the motion of LAA.

### Embodiment 15

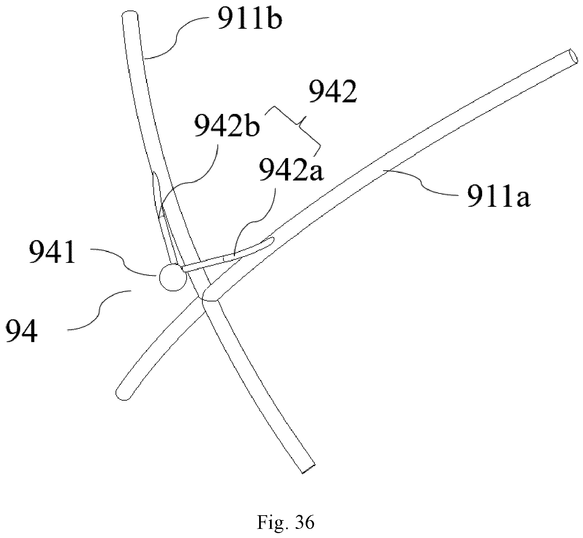

With reference to FIG. 36, the medical device of this embodiment is substantially identical to those of Embodiments 13-14, and the identical parts will not be described herein. The main difference lies in that the anchoring unit 24 includes a connecting unit 942 and at least one anchoring unit 941 which is connected to a plurality of supporting wires 911 via the connecting unit 942. When the fixing part is in a radially compressed state, the anchoring unit 941 is positioned at a medial side of the mesh support structure 11, or the anchoring unit 941 is positioned at least partially in a gap between adjacent supporting wires 911.

With reference to FIG 11, the anchoring unit 941 is connected to a first supporting wire 911a via a first connecting unit 942a and connected to a second supporting wire 911b via a second connecting unit 942b, and an included angle of 10°-90° is formed between the first connecting unit 942a and the second connecting unit 942b. The connection of the first connecting unit 942a with the first supporting wire 911a, and the connection of the second connecting unit 942b with the second supporting wire 911b may be achieved by one or more of welding, hot melting, entanglement, and bonding.

The first connecting unit 942a and second connecting unit 942b described above may have an integral structure and run through the anchoring unit 941. Alternatively, the first connecting unit 942a and the second connecting unit 942b may have a split structure. The anchoring unit 941 is fixedly or movably connected to the first connecting unit 942a and the second connecting unit 942b. In other embodiments, a plurality of anchoring units 941 may be arranged in series on the first connecting unit 942a and the second connecting unit 942b.

During radial compression of the medical device (see FIG. 33), the anchoring unit 941 is pressed into a gap between the first supporting wire 911a and the second supporting wire 911b and moved to the medial side of the mesh support structure 11. Alternatively, the anchoring unit 941 is only partially pressed into the gap between the first supporting wire 911a and the second supporting wire 911b during radial compression of the medical device. Thus, the radial size of the medical device can be reduced after compression.

The first connecting unit 942a and the second connecting unit 942b described above may both be made of a hard metal (such as nickel-titanium), a hard polymer material, etc., or may be made of a flexible and soft material, such as a rod, wire, thread, or elastic rope structure formed by a soft polymer material, an inorganic material, or a flexible metal, etc.

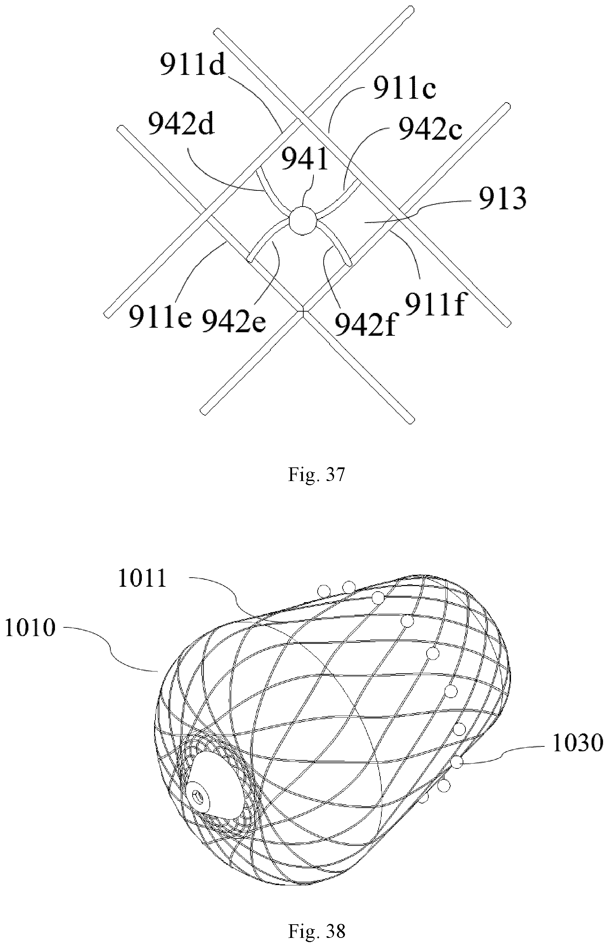

With reference to FIG 37, in other embodiments, the anchoring unit 941 may be further connected to a third supporting wire 942c via a third connecting unit 911c, connected to a fourth supporting wire 942d via a fourth connecting unit 911d, connected to a fifth supporting wire 942e via a fifth connecting unit 911e, and connected to a sixth supporting wire 942f via a sixth connecting unit 911f. The connection methods include one or more of welding, hot melting, entanglement, and bonding.

The third connecting unit 911c, the fourth connecting unit 911d, the fifth connecting unit 911e, and the sixth connecting unit 911f described above cannot all be made of a hard metal, a hard polymer material, etc., and at least one of the four units needs to be made of a flexible and soft material, such as a rod, wire, thread, or elastic rope structure formed by a soft polymer material, an inorganic material, or a flexible metal, etc. Therefore, it is ensured that during radial compression of the medical device, the anchoring unit 941 is pressed into a mesh 113 enclosed by the third connecting unit 911c, the fourth connecting unit 911d, the fifth connecting unit 911e, and the sixth connecting unit 911f and moved to the medial side of the mesh support structure 11. Alternatively, the anchoring unit 941 is only partially pressed into the mesh 113 during radial compression of the medical device. The anchoring unit 941 is connected to two or more supporting wires 911 in multiple directions via two or more connecting units 942, such that the stress on the anchoring unit 941 is balanced, and it is ensured that the anchoring unit 941 is pressed into the mesh 113 during radial compression of the medical device.

### Embodiment 16

With reference to FIG. 38, the medical device of this embodiment is substantially identical to those of Embodiments 13-15, and the identical parts will not be described herein. The main difference lies in that in this embodiment, the medical device has a plug-type structure, including a fixing part 1010, a film (not shown), and at least one anchoring member 1030. The fixing part 1010 includes a mesh support structure 1011, the film may cover only a proximal portion of the mesh support structure 1011 or fully cover the entire mesh support structure 1011, and the film may be arranged inside the mesh support structure 1011 or outside the mesh support structure 1011. The anchoring member 1030 may be connected to the mesh support structure 1011 and/or the film. The specific shape and structure of the anchoring member 1030 and the connection method thereof with the fixing part 30 may be obtained with reference to Embodiments 13-15, which will not be described in detail herein.

In this embodiment, the fixing part 1010 described above has not only the effect of anchoring the medical device, but also the occlusion effect. In other embodiments, there is another medical device including an integral mesh structure having two ends forming a fixing disc and a sealing disc, separately, and a waist connecting the fixing disc and the sealing disc. An anchoring member 1030 may also be provided on the medical device with reference to any of the embodiments described above.

It is to be noted that the protrusion is not the only manifestation of the anchoring unit, and the anchoring unit may be configured in a variety of structures, such as annular, umbrella-shaped, disc-shaped, and radial, as long as it is ensured that the anchoring unit can be adaptively clamped in or extend into the gap in the human tissue or abut against the human tissue. In addition, the technical features of the embodiments described above can be combined in any combination, and can also be applied together to various LAA occluders described above as well as LAA occluders with similar structures. In order to make the description concise, not all the possible combinations of the technical features in the embodiments above are described. However, the combinations of these technical features shall be considered as falling with the scope of the description as long as there is no contradiction therein.

In addition to application of various embodiments in LAA described above, it is to be further noted that the medical device can be used in a number of different environments, for example, in intracranial aneurysm operations, where an occluder can be used for occlusion at the site of the aneurysm, and that the anchoring members of the embodiments above may also be used in conjunction with the occluder. In addition, it is to be noted that the anchoring members of the embodiments above can be applied to various implants such as a stent or a filter, in addition to application in a medical device, i.e., the structures of the anchoring members are not affected by the structure of the medical device itself.

The embodiments described above express only a few implementations of the present invention which are described in detail and should not therefore be construed as limiting the scope of the present invention. It is to be noted that a person of ordinary skill in the art would be able to make several variations and improvements without departing from the concept of the present invention, which fall within the protection scope of the present invention. Therefore, the protection scope of the present invention shall be subject to the appended claims.

## Claims

1. A medical device, comprising a fixing part configured to fix the medical device at a predetermined position, wherein the fixing part is provided with at least one anchoring member configured to abut against a target cavity after the medical device is implanted at the predetermined position, so as to anchor the medical device at the predetermined position.

2. The medical device of claim 1, wherein the anchoring member comprises at least one anchoring unit.

3. The medical device of claim 2, wherein the anchoring member further comprises a connecting unit.

4. The medical device of claim 3, wherein the fixing part comprises a cover membrane, and the connecting unit connects the anchoring unit to the cover membrane.

5. The medical device of claim 3, wherein the fixing part comprises a mesh support structure, and the connecting unit connects the anchoring unit to the mesh support structure.

6. The medical device of claim 3, wherein the fixing part comprises a supporting rod, and the connecting unit connects the anchoring unit to the supporting rod.

7. The medical device of claim 4, wherein the anchoring unit is positioned between the fixing part and the cover membrane, and the cover membrane at a position of the anchoring unit protrudes in a direction facing away from an axis of the medical device.

8. The medical device of claim 5, wherein the mesh support structure comprises a supporting wire, the connecting unit is connected to the supporting wire by entanglement, and the connecting unit is fixedly connected to the supporting wire, or the connecting unit is slidable with respect to the supporting wire along a length direction of the supporting wire.

9. The medical device of claim 8, wherein a limit unit is further provided on the supporting wire to which the connecting unit is connected so as to limit a sliding range of the connecting unit on the supporting wire when the connecting unit is slidable with respect to the supporting wire along the length direction of the supporting wire.

10. The medical device of claim 5, wherein the mesh support structure comprises a plurality of supporting wires, the anchoring unit is connected to the plurality of supporting wires by the connecting unit, and when the fixing part is in a radially compressed state, the anchoring unit is positioned at a medial side of the mesh support structure, or the anchoring unit is positioned at least partially in a gap between adjacent supporting wires.

11. The medical device of claim 5, wherein a plurality of anchoring units are provided on the fixing part, and the plurality of anchoring units are positioned on a plurality of cross-sections of the fixing part when the fixing part is in a deployed state; and/or the plurality of anchoring units are positioned on a plurality of cross-sections of the fixing part when the fixing part is in a radially compressed state.

12. The medical device of any one of claims 3-11, wherein the anchoring member comprises a connecting rod and a connecting block, a first channel is arranged on the fixing part which runs through medial and lateral sides of the fixing part, one end of the connecting rod is connected to the anchoring unit, and the other end is connected to the connecting block after passing through the first channel.

13. The medical device of any one of claims 3-11, wherein the anchoring unit is rotatably connected to the connecting unit.

14. The medical device of any one of claims 3-11, wherein the connecting unit comprises an axially retractable rod having one end connected to the anchoring unit and the other end fixedly connected to the fixing part.

15. The medical device of any one of claims 3-11, wherein the fixing part is provided with a receiving cavity that receives or partially receives the anchoring unit when the medical device is in a compressed state.

16. The medical device of claim 15, wherein the receiving cavity comprises a first cavity that at least partially receives the anchoring unit when the medical device is in the compressed state and a second cavity that at least partially receives the connecting unit when the medical device is in the compressed state.

17. The medical device of any one of claims 3-11, wherein the anchoring unit extends obliquely outward in a natural state, and an opening angle of the anchoring unit with respect to the fixing part in the natural state is 20°-60°.

18. The medical device of any one of claims 3-11, wherein the anchoring member further comprises a flexible connecting unit, at least one hole is arranged on the fixing part which runs through medial and lateral sides of the fixing part, one end of the flexible connecting unit is movably fixed to the fixing part after passing through the hole, and the anchoring unit is fixed to the flexible connecting unit.

19. The medical device of any one of claims 3-11, wherein the fixing part comprises a plurality of anchoring members, at least one of the anchoring members having a different opening angle and/or a different horizontal position from anchoring members adjacent thereto.

20. The medical device of any one of claims 3-11, wherein the anchoring unit is provided with micro-barbs.
